(19) Europäisches Patentamt
European Patent Office
Office européen des brevets

(11) Publication number: **0 080 271**
**A1**

(12) # EUROPEAN PATENT APPLICATION

(21) Application number: **82305713.8**

(22) Date of filing: **27.10.82**

(51) Int. Cl.³: **C 07 D 209/28**, A 61 K 31/405

(30) Priority: **27.10.81 JP 172289/81**

(43) Date of publication of application: **01.06.83**
**Bulletin 83/22**

(84) Designated Contracting States: **AT BE CH DE FR GB IT LI NL SE**

(71) Applicant: **TEIKOKU KAGAKU SANGYO K.K., 1-18, Kitahorie 1-chome, Nishi-ku Osaka (JP)**

(72) Inventor: **Nakajima, Isao, 4-4-17 Hattorihonmachi, Toyonaka-shi Osaka-fu (JP)**
Inventor: **Adachi, Tsutomu, 2-7-3 Nishidai, Itama-shi Hyogo-ken (JP)**
Inventor: **Takeda, Shuichi, 2-16-14 Ohasuhigashi, Higashiosaka-shi Osaka-fu (JP)**
Inventor: **Tanaka, Ikuo, 1352 Aomadani, Mino-shi Osaka-fu (JP)**

(74) Representative: **Perry, Robert Edward et al, GILL JENNINGS & EVERY 53-64 Chancery Lane, London WC2A 1HN (GB)**

(54) **Indomethacin phenyl ester derivatives, their use, and compositions containing them.**

(57) Indomethacin derivatives of the formula

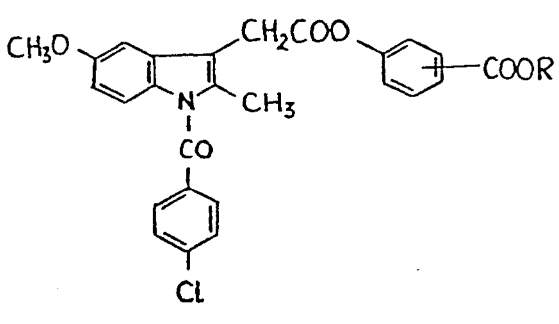

I

wherein R is hydrogen, lower alkyl, aralkyl or substituted phenyl can have anti-inflammatory activity, without causing ulceration.

EP 0 080 271 A1

# INDOMETHACIN PHENYL ESTER DERIVATIVES, THEIR USE, AND COMPOSITIONS CONTAINING THEM

The present invention relates to indomethacin phenyl ester derivatives, their use, and compositions containing them.

Indomethacin is well known as an anti-inflammatory agent, but its use is limited by its tendency to cause ulcers. Various indomethacin derivatives are known, including 2-hydroxycarbonylphenyl 1-(p-chlorobenzoyl)-5-methoxy-2-methylindole-3-acetate. Ceskoslovenska Farmacie 26 (1977) 6277 reports that this compound is not useful, because of its hydrolysis by serum esterase.

According to the present invention, compounds for pharmaceutical, and particularly anti-inflammatory, use, have the formula

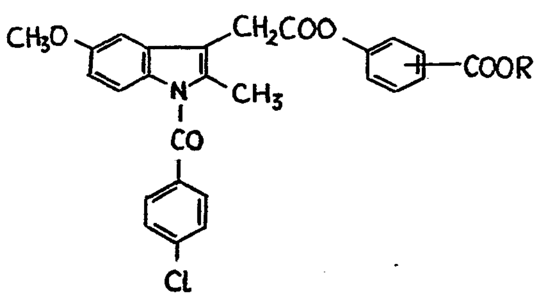

I

wherein R is hydrogen, lower (e.g. $C_{1-8}$) alkyl, aralkyl or substituted phenyl. The compounds of formula I in which R is alkyl, aralkyl or substituted phenyl are novel compounds of the invention. A pharmaceutical composition of the invention comprises a compound of formula I in association with a physiologically acceptable excipient.

The compounds of the invention can have good anti-inflammatory activity *in vivo*, but without the undesirable side-effects associated with indomethacin. In particular, the known indomethacin ester described

above shows high ulceration inhibition.

Compounds of formula I may be prepared by reacting indomethacin, or a reactive derivative thereof, in a suitable solvent, with an appropriate hydroxybenzoate. If desired, a condensation agent may be present. Again, if desired, the product may be subjected to hydrogenolysis.

Suitable reactive derivatives of indomethacin include acid halides and activated esters thereof. Examples of suitable hydroxybenzoates are the lower alkyl, e.g. methyl, ethyl or t-butyl, esters, aralkyl, e.g. benzyl, phenethyl or anisyl, esters, and substituted phenyl, e.g. benzyloxycarbonylphenyl, esters such as hydroxybenzoyloxybenzoic acid. It is preferred that the product should have the COOR group at the ortho-position relative to the indomethacin radical.

If the reaction involves the use of an indomethacin acid halide, it is preferred that an amine is also used, in order to neutralise the hydrogen halide which is produced. Suitable amines are tertiary amines such as triethylamine, pyridine and dimethylaniline.

The products of the invention may exist in crystalline form. For use, they may be compounded into compositions comprising conventional excipients which are preferably solids or sterile liquids. Such compositions may be provided in unit dosage, i.e. discrete, form. The compound may comprise a minor proportion of the composition, with the excipient (which may comprise a mixture of components) constituting the major proportion, by weight.

The following Examples illustrate how compounds of formula I may be prepared.

Example 1

2-Benzyloxycarbonylphenyl 1-(p-chlorobenzoyl)-5-methoxy-2-methylindole-3-acetate

35.8 g indomethacin and 22.9 g benzyl salicylate were dissolved in 200 ml pyridine. To this solution were added 2.7 g dicyclohexylcarbodiimide, and the mixture was reacted with stirring at room temperature for 15 hours. Volatile materials were removed under reduced pressure, and the residue was treated with 300 ml chloroform and 100 ml water,and thereafter acidified with hydrochloric acid. After removing the undissolved substances, the filtrate was kept standing and allowed to separate into two layers. The separated organic phase was washed with water, dried and concentrated. The residuce was treated with ethyl acetate and kept in a refrigerator. The precipitated materials were removed, the filtrate concentrated, and the residue was crystallised from a mixture of ether and n-hexane to obtain 37 g of the title compound, m.p. 123.5-125°C.

IR spectrum (in mineral oil) ν, $cm^{-1}$ : 1760, 1720, 1665(>C=O).

$^1H$-NMR spectrum (in $CDCl_3$) δ, ppm : 2.38 (s, 3H), 3.82 (s, 3H), 3.87 (s, 2H), 5.30 (s, 2H), 6.58-8.16 (m, 16H).

Elementary analysis for $C_{33}H_{26}ClNO_6$ :

Calcd. : C, 69.78; H, 4.61; N, 2.47.

Found : C, 69.81; H, 4.45; N, 2.49.

Example 2

2-Ethoxycarbonylphenyl 1-(p-chlorobenzoyl)-5-methoxy-2-methylindole-3-acetate

The title compound was obtained by the reaction of indomethacin (17.9 g), ethyl salicylate (8.3 g) and dicyclohexylcarbodiimide (12.4 g) according to the procedures of Example 1. Yield: 11.3 g, m.p. 80-85°C.

IR spectrum (in mineral oil) ν, $cm^{-1}$ : 1765, 1720, 1660(>C=O).

$^1H$-NMR spectrum ($CDCl_3$) δ, ppm : 1.33 (T, 3H), 2.41 (s, 3H), 3.80 (s, 3H), 3.98 (s, 2H), 4.29 (q, 2H), 6.57-8.03, 7.33 (m,s, 11H).

Elementary analysis for $C_{28}H_{24}ClNO_6$ :

Calcd. : C, 66.47; H, 4.78; N, 2.77.
Found : C, 66.87; H, 4.79; N, 2.71.

$LD_{50}$ of this compound, determined by means of oral administration in mice of the stock ddy, was 200 mg/kg.

Example 3

2-t-Butoxycarbonylphenyl 1-(p-chlorobenzoyl)-5-methoxy-2-methlindole-3-acetate

17.4 g indomethacin and 13.5 g t-butyl salicylate were dissolved in 100 ml pyridine. To this solution were added 12.0 dicyclohexylcarbodiimide, and the reaction mixture was stirred at room temperature for 20 hours. After removing the undissolved substances, the filtrate was concentrated, diluted with 150 ml chloroform and 50 ml water, and acidified with hydrochloric acid to pH 3. The undissolved substances were removed, and the separated chloroform layer was washed with water, dried and concentrated. The residue was subjected to column chromatography to obtain 15 g of the title compound, m.p. 100-101.5$^oC$.

IR spectrum (in mineral oil) ν, $cm^{-1}$ : 1760, 1720, 1690 (>C=O).

$^1H$-NMR spectrum ($CDCl_3$) δ, ppm : 1.50 (s, 9H), 3.78 (s, 3H), 3.94 (s, 2H), 6.60-7.84 (m, 11H).

Elementary analysis for $C_{30}H_{28}ClNO_6$ :

Calcd. : C, 67.48; H, 5.29; N, 2.62.
Found : C, 67.54; H, 5.25; N, 2.59.

Example 4

2-(2-Benzyloxycarbonylphenyl)oxycarbonylphenyl 1-(p-chlorobenzoyl)-5-methoxy-2-methylindole-3-acetate

12.8 g indomethacin and 12.5 g benzyl salicylate were dissolved in 40 ml pyridine and 8.2 g dicyclohexylcarbodiimide were added to the resultant solution. The reaction mixture was stirred at room temperature for 30 hours. After removing the undissolved substances, the filtrate was concentrated, diluted with chloroform (70 ml) and water (30 ml), and acidified with hydrochloric acid to pH 3. The undissolved substances were removed, the chloroform layer was separated, washed with water and dried. After removing the solvent, 21.53 g of the title compound, m.p. 57.5-60°C, were obtained from the residue by chromatographic separation.

IR spectrum (in mineral oil) ν, $cm^{-1}$ : 1770, 1740, 1720, 1680 (>C=O).

$^1H$-NMR spectrum ($CDCl_3$) δ, ppm : 2.30 (s, 3H), 3.60 (s, 3H), 3.90 (s, 2H), 5.16 (s, 2H), 6.46-8.23 (m, 20H).

Elementary analysis for $C_{40}H_{30}ClNO_8$ :

Calcd. : C, 69.82; H, 4.39; N, 2.04.
Found : C, 69.99; H, 4.31; N, 2.00.

Example 5

2-(2-Hydroxycarbonylphenyl)oxycarbonylphenyl 1-(p-chlorobenzoyl)-5-methoxy-2-methylindole-3-acetate

14.3 g of the compound of Example 4 were hydrogenated in N,N-dimethylformamide in the presence of palladium-carbon. After removing the catalyst, the filtrate was poured into ice-water and the precipitated product was filtered, to obtain 8.15 g of the title compound, m.p. 116-120°C.

IR spectrum (in mineral oil) ν, $cm^{-1}$ : 1760, 1730, 1700, 1670 (>C=O).

$^1H$-NMR spectrum ($CDCl_3$) δ, ppm : 2.30 (s, 3H),

3.60 (s, 3H), 3.90 (s, 2H), 5.98 (brs. 1H, substitutable with $D_2O$), 6.60-8.30 (m, 15H).

Elementary analysis for $C_{33}H_{24}ClNO_8$ :

Calcd. : C, 66.28; H, 4.05; N, 2.34.

Found : C, 66.41; H, 3.91; N, 2.29.

Example 6

2-(2-Hydroxycarbonylphenyl)oxycarbonylphenyl 1-(p-chlorobenzoyl)-5-methoxy-2-methylindole-3-acetate

35.8 g indomethacin and 13.1 g thionyl chloride were reacted in 150 ml methylene chloride, while refluxing, for 30 minutes. After removing the solvent, the residue was diluted with 100 ml methylene chloride, and then with a solution of 38.3 g benzyl salicylsalicylate and 8.7 g pyridine in 50 ml methylene chloride. The mixture was reacted at room temperature overnight. The reaction mixture was washed with 100 ml 5% hydrochloric acid solution and then with 100 ml water, and the solvent was removed. 300 ml dimethylformamide were added to the residue and the solution was subjected to hydrogenolysis in the presence of palladium-carbon. After removing the catalyst, the filtrate was poured into water, the precipitated product was filtered, washed with ethyl alcohol, and dried to obtain 53.3 g of the title compound (yield 89.1%). A part of the product was recrystallised from acetonitrile to give product, m.p. 112-116$^{o}$C.

Example 7

2-(2-Hydroxycarbonylphenyl)oxycarbonylphenyl 1-(p-chlorobenzoyl)-5-methoxy-2-methylindole-3-acetate

3.58 g indomethacin and 1.31 g thionyl chloride were reacted in chloroform at 45$^{o}$C for 30 minutes. The reaction mixture was added drop-wise to a solution of 3.87 g salicylsalicylate, 3.16 g pyridine and 10 ml chloroform, and the thus-obtained mixture was reacted at room temperature for 5 hours. The reaction mixture

was washed with 20 ml of each of 3% hydrochloric acid and water, and the solvent was removed. The residue was diluted with 5 ml methanol, and the precipitated crystals were separated to obtain 3.7 g of the title compound (yield 61.9%). A part of the product was recrystallised from trichlorethylene to give product, m.p. 133-136°C.

Example 8

2-(2-Hydroxycarbonylphenyl)oxycarbonylphenyl 1-(p-chlorobenzoyl)-5-methoxy-2-methylindole-3-acetate

10.7 g indomethacin and 4.3 g thionyl chloride were reacted in toluene at 50°C for 30 minutes. After removing the solvent, 100 ml fresh toluene were added, to give a clear solution. This solution was added to a solution obtained by the reaction of 8.5 g salicyl salicylate , 3.9 g trimethylchlorosilane and 9.5 g pyridine in 80 ml toluene, and the mixture was reacted at room temperature for 5 hours. After washing with 100 ml 5% hydrochloric acid and 100 ml water, the solvent was removed, the residue was diluted with 80 ml methanol, and the precipitated crystals were filtered to obtain 15.0 g of the title compound (yield 83.6%). A part of the product was recrystallised from ethanol to give product, m.p. 148-150°C.

Pharmacological tests have been conducted in order to assess the activity of the indomethacin phenyl ester derivatives of this invention. Firstly, in order to assess anti-inflammatory activity, the inhibition of edema induced by carrageenin in rat was studied. Various compounds (listed in Table 1) were administered orally to a group of rats just 1 hour before a carrageenin injection, and the hind paw volume was measured 3 hours after injection. The results are shown in Table 1.

TABLE 1

| Compound | dose (mg/kg (mM)) | edema (%) | inhibition (%) |
|---|---|---|---|
| Control | — | 81.0 ± 6.1 | — |
| indomethacin | 5.4 (0.015) | 54.9 ± 6.5 | 32.2 |
| | 10.8 (0.030) | 37.7 ± 7.2 | 53.5 |
| Example 2 | 7.6 (0.915) | 53.3 ± 8.7 | 31.8 |
| | 15.2 (0.030) | 52.9 ± 3.6 | 34.7 |
| Example 3 | 8.0 (0.015) | 52.0 ± 7.5 | 35.8 |
| | 16.0 (0.030) | 52.9 ± 3.6 | 34.7 |
| Example 5 | 9.0 (0.015) | 46.4 ± 4.8 | 42.7 |
| | 18.0 (0.030) | 41.4 ± 5.3 | 48.7 |
| Indomethacin salicylic ester | 7.2 (0.015) | 41.0 ± 3.5 | 49.4 |

Secondly, in order to assess ulceration activity, various compounds (listed in Table 2) were administered orally to a group of rats. The animals were sacrificed 5 hours after administration, and the ulcer index was determined. The results are shown in Table 2. Compounds were also administered orally to a group of rats for 1 week, and the number of intestinal ulcers was determined. The results are shown in Table 3. The same compounds were administered orally to a group of rats, the ulcer index being determined by observing the stomach 3 hours after administration. The results are shown in Table 4.

TABLE 2

| Compound | dose (mg/kg (mM)) | ulcer index (mm) |
|---|---|---|
| Indomethacin | 10.8 (0.030)<br>21.5 (0.060) | 5.9 ± 2.5<br>57.9 ± 6.6 |
| Example 1 | 17.6 (0.030) | 0.1 ± 0.1 |
| Example 2 | 15.2 (0.030) | 0.2 ± 0.1 |
| Example 3 | 16.0 (0.030) | 0.2 ± 0.1 |
| Example 4 | 20.6 (0.030) | 0.4 ± 0.3 |
| Example 5 | 18.0 (0.030) | 0.6 ± 0.3 |

TABLE 3

| Compound | dose (mg/kg (μM)) | number of ulcers | inhibition (%) |
|---|---|---|---|
| Indomethacin plus salicyl salicylate | 5.0 (15)<br>3.6 (15) | 30.4 ± 6.0 | |
| Example 5 | 8.4 (15) | 0.5 ± 2.0 | 98.4 |

TABLE 4

| Compound | dose (mg/kg (mM)) | Ulcer index | inhibition (%) |
|---|---|---|---|
| Indomethacin plus salicyl salicylate | 20.0 (0.056)<br>14.4 (0.056) | 26.4 ± 7.4 | |
| Example 5 | 33.6 (0.056) | 3.7 ± 1.3 | 86.0 |

Each compound listed in Table 5 was administered orally to a group of 12 rats for 5 days, and the number of intestinal ulcers was determined. The results are shown in Table 5. The degree of ulceration was calculated as a percentage of number of uncleared surviving animals with respect to the number of surviving animals.

TABLE 5

| Compound | daily dose (mg/kg (μM)) | number of survival animals | number of intestinal ulcers | ulceration (%) |
|---|---|---|---|---|
| Indomethacin | 10 (28) | 0 | | |
| Acemethacin | 11.6 (28) | 1 | 11.0 | 100 |
| Indomethacin salicylic ester | 13.4 (28) | 8 | 5.0 ± 0.4 | 100 |
| Indomethacin salicylsalicylic ester | 16.7 (28) | 11 | 2.4 ± 0.6 | 75 |

Each compound listed in Table 6 was administered orally to a group of 10 rats and the ulcer index was determined by observing the stomach 24 hours after administration. The results are shown in Table 6.

TABLE 6

| Compound | dose (mg/kg (mM)) | Ulcer index (mm) | inhibition (%) |
|---|---|---|---|
| Indomethacin salicylic ester | 28.7 (0.060) | 7.1 ± 1.6 | 73.2 |
| Indomethacin | 21.6 (0.060) | 26.5 ± 6.2 | |
| Equimolar mixture of indomethacin and its salicylic ester | 29.8 (0.060) | 24.8 ± 5.6 | 64 |

CLAIMS

1. A compound, for pharmaceutical use, of the formula

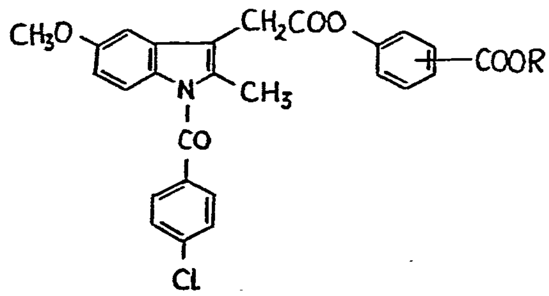

I

wherein R is hydrogen, lower alkyl, aralkyl or substituted phenyl.

2. A compound of formula I as defined in claim 1, wherein R is lower alkyl, aralkyl or substituted phenyl.

3. A compound as claimed in claim 1 or claim 2, wherein the COOR group is at the ortho-position on the benzene ring to which it is attached.

4. 2-(2-Hydroxycarbonylphenyl)oxycarbonylphenyl 1-(p-chlorobenzoyl)-5-methoxy-2-methylindole-3-acetate.

5. A compound as claimed in any preceding claim, for anti-inflammatory use.

6. A pharmaceutical composition comprising a compound as claimed in any preceding claim, in association with a physiologically acceptable excipient.

CLAIMS (FOR AUSTRIA)

1. A process for preparing a compound of the formula

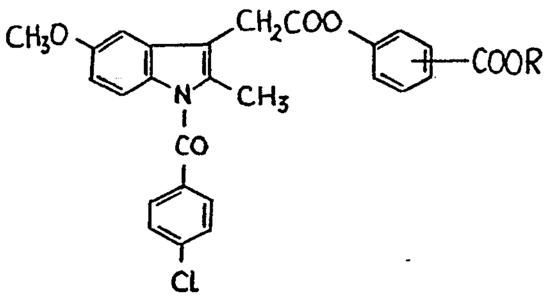

I

wherein R is hydrogen, lower alkyl, aralkyl or substituted phenyl, which comprises reacting indomethacin, or a reactive derivative thereof, with a phenol carrying a COOR' substituent, R' being lower alkyl, aralkyl or substituted phenyl; and, if desired, hydrogenating the product.

2. A process according to claim 1, in which the COOR substituent is at the ortho-position of the benzene ring to which it is attached.

3. A process according to claim 1, in which the product is 2-(2-hydroxycarbonylphenyl)oxycarbonylphenyl 1-(p-chlorobenzoyl)-5-methoxy-2-methylindole-3-acetate.

0080271

European Patent Office

# EUROPEAN SEARCH REPORT

Application number

EP 82 30 5713

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| | --- | | C 07 D 209/28<br>A 61 K 31/405 |
| A | Chemical Abstracts vol. 95, no. 1, 6 July 1981, Columbus, Ohio, USA, L. FISNEROVA et al. "Esters of 1-(p-chlorobenzoyl)-5-methoxy-2-methyl-3-indolylacetic acid", page 667, right-hand column, abstract no. 7061r & CS-A-184941 | 1,5 | |
| | --- | | |
| A | Chemical Abstracts vol. 88, no. 9, 27 February 1978, Columbus, Ohio, USA, L. FISNEROVA et al. "Pharmacologically interesting indomethacin derivates" page 373, right-hand column, abstract no. 62247m & Cesk. Farm. vol. 26, no. 6, 1977, pages 227-231 | 1,5 | |
| | ----- | | TECHNICAL FIELDS SEARCHED (Int. Cl. 3) |
| | | | A 61 K 31/405<br>C 07 D 209/28 |

The present search report has been drawn up for all claims

| Place of search | Date of completion of the search | Examiner |
|---|---|---|
| BERLIN | 24-01-1983 | PHILLIPS N.G.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document